# EUROPEAN PATENT APPLICATION

(11) **EP 3 305 307 A1**
(43) Date of publication of application: **11.04.2018**
(21) Application number: 16803251.4
(22) Date of filing: 27.05.2016
(51) Int. Cl.: A61K 35/36, A61P 17/00, A61P 17/02, G01N 33/15

(54) **PLURIPOTENT STEM CELL MIGRATION PROMOTER**

(30) Priority: 29.05.2015 JP 2015110656
(71) Applicant: Nippon Zoki Pharmaceutical Co., Ltd., Osaka-shi, Osaka 541-0046 (JP)
(72) Inventor: DEZAWA, Mari, Sendai-shi Miyagi 980-8575 (JP); OHTSU, Mieko, Akita-shi Akita 010-0001 (JP)
(74) Representative: Hoffmann Eitle
(86) International application number: PCT/JP2016/065735
(87) International publication number: WO 2016/194816

(57) **Abstract**

An object of the present invention is to provide, for example, an agent for promoting migration of pluripotent stem cells containing an extract from inflamed tissues inoculated with vaccinia virus. The present invention has proven that an extract from inflamed tissues inoculated with vaccinia virus promotes migration of Muse cells. Thus, the present invention provides, for example, an agent for promoting migration of pluripotent stem cells for various diseases to which migration of pluripotent stem cells may have an advantageous effect.

## Description

### [Technical Field]

The present invention relates to a novel medical use of an extract from inflamed tissues inoculated with vaccinia virus (hereinafter, it will be sometimes referred to as "the present extract") and, more particularly, it relates to an agent for promoting migration of pluripotent stem cells containing an extract from inflamed tissues inoculated with vaccinia virus as an active ingredient.

Stem cells are a type of cells that have self-proliferation ability and pluripotency. Known stem cells include embryonic stem cells (ES cells) and induced pluripotent stem cells (iPS cells). ES cells have ethical issues because they are obtained by destroying a fertilized egg. In addition, ES cells can cause rejection because the donor of the fertilized egg is not the patient themselves. iPS cells also have problems, as follows: the process of establishing iPS cells is complicated including introducing a specific gene and a specific compound into a somatic cell; and introducing artificial reprogramming factors can cause gene damage or leave some undifferentiated cells and may eventually lead to tumorigenesis.

Meanwhile, a living organism naturally has stem cells called mesenchymal stem cells, which are known to be capable of differentiating into bone, cartilage, adipocytes, neurons, and skeletal muscle, for example. This differentiation ability is under research for developing treatment of bone and joint diseases, spinal injury, Parkinson's disease, and diabetes, for example. It is known that mesenchymal stem cells are obtainable from such tissues as bone marrow tissue, adipose tissue, placental tissue, umbilical cord tissue, and dental pulp tissue. Mesenchymal stem cells are an assembly of various cells and therefore the therapeutic effect of each assembly may greatly vary, which is unfavorable.

The inventors of the present invention conducted research and discovered a type of pluripotent stem cells (Multilineage-differentiating Stress Enduring cells, or Muse cells) contained in the mesenchymal cell fraction and obtainable without any complicated induction process such as gene transfer. Muse cells express a surface antigen called SSEA-3 (Stage-Specific Embryonic Antigen-3). It was found that Muse cells are present in mesenchymal tissue of a living organism, such as skin, bone marrow, and lipid; and when transplanted in various mouse models (immune deficient with no rejection displayed to human cells) of spinal cord injury, liver injury, gastrocnemius injury, and other diseases, Muse cells accumulate at a damaged site and differentiate into cells of the damaged tissue (WO 2011/007900). In addition, the inventors of the present invention demonstrated the following: Muse cells transplanted in a rabbit model of myocardial infarction accumulate at the infarct and make the infarct smaller (WO 2014/027474); and sphingosine-1-phosphate (S1P) and the like enhance chemotactic ability of Muse cells and guide migration of Muse cells from mesenchymal tissue to a damaged site (WO 2014/133170).

The extract from inflamed tissues inoculated with vaccinia virus (the present extract) containing in the agent for promoting migration of pluripotent stem cells or a preparation containing the present extract of the present invention is disclosed to have the following effects: an analgesic effect, sedative effect, anti-stress effect and anti-allergic effect (see Patent Document 1); an immunostimulating effect, anti-cancer effect and cirrhosis inhibitory effect (see Patent Document 2); a treatment effect against idiopathic thrombocytopenic purpura (see Patent Document 3); a treatment effect against postherpetic neuralgia, brain edema, dementia, spinocerebellar degeneration and the like (see Patent Document 4); a treatment effect against Raynaud syndrome, diabetic neuropathy, sequelae of subacute myelo-optico-neuropathy and the like (see Patent Document 5); a kallikrein production inhibitory effect and peripheral circulatory disorder improving effect (see Patent Document 6); a bone atrophy improving effect (see Patent Document 7); a nitric oxide production inhibitory effect effective for the treatment of sepsis and endotoxic shock (see Patent Document 8); a treatment effect against osteoporosis (see Patent Document 9); a treatment effect against AIDS based on a Nef action inhibitory effect and chemokine production inhibitory effect (see Patent Documents 10 and 11); a treatment effect against ischemic disorders such as cerebral infarction (see Patent Document 12); a treatment effect against fibromyalgia syndrome (see Patent Document 13); a treatment effect against infections (see Patent Document 14); prophylactic or alleviating effect for a peripheral nerve disorder induced by an anti-cancer agent (see Patent Document 15) ; a treatment effect against chronic prostatitis, interstitial cystitis and/or urination disorders (see Patent Document 16) ; an effect of promoting production of neurotrophic factor such as BDNF (see Patent Document 17); an effect of promoting the synthesis of collagen and proteoglycan in chondrocytes (see Patent Document 18) and the like. However, it is not known that the present extract or the preparation containing the present extract is effective for promoting migration of pluripotent stem cells.

### [Prior Art Documents]

### [Patent Documents]

Patent Document 1: Japanese Patent Laid-Open No. Sho-53-101515
Patent Document 2: Japanese Patent Laid-Open No. sho-55-87724
Patent Document 3: Japanese Patent Laid-Open No. Hei-1-265028
Patent Document 4: Japanese Patent Laid-Open No. Hei-1-319422
Patent Document 5: Japanese Patent Laid-Open No. Hei-2-28119
Patent Document 6: Japanese Patent Laid-Open No. Hei-7-97336
Patent Document 7: Japanese Patent Laid-Open No. Hei-8-291077
Patent Document 8: Japanese Patent Laid-Open No. Hei-10-194978
Patent Document 9: Japanese Patent Laid-Open No. Hei-11-80005
Patent Document 10: Japanese Patent Laid-Open No. Hei-11-139977
Patent Document 11: Japanese Patent Laid-Open No. 2000-336034
Patent Document 12: Japanese Patent Laid-Open No. 2000-16942
Patent Document 13: International Publication No. WO 2004/039383
Patent Document 14: Japanese Patent Laid-Open No. 2004-300146
Patent Document 15: International Publication No. WO2009/028605
Patent Document 16: International Publication No. WO2011/111770
Patent Document 17: International Publication No. WO2011/162317
Patent Document 18: International Publication No. WO2012/051173

### [Summary of the Invention]

### [Problems to be solved by the invention]

An object of the present invention is to provide an agent for promoting migration of pluripotent stem cells containing the present extract and the like.

### [Means for Solving the Problems]

The present inventors have conducted intensive studies for a drug therapy of damage for which effective therapeutic method has been demanded and, as a result, they have found that the present extract shows excellent effect of promoting migration of pluripotent stem cells and achieved the present invention.

### [Advantages of the Invention]

Since the present extract has an excellent pharmacological action that it promotes migration of pluripotent stem cells and moreover, the preparation containing the present extract is a safe medicinal agent having little problem such as side effect, the present invention is very highly useful.

In the present invention, damage refers to a systemic or local damage of a living organism caused by an internal and/or external factor. The damage in the present invention includes various traumas as well as various conditions such as infarction, degenerative changes, and tissue destruction. Examples of the damaged site in the present invention include, but not limited to, various tissues of a body such as brain, nerve, kidneys, pancreas, liver, heart, skin, bone, and cartilage. Examples of the factor that causes the damage include, but not limited to, physical external forces (such as an accident, a burn, and radiation exposure), ischemic heart diseases such as myocardial infarction, various inflammations, diabetes, various infectious diseases, autoimmune diseases, tumors, toxic exposure, and neurodegenerative diseases.

### [Brief Description of Drawings]

Fig. 1 shows stained images of Muse cells that have passed through a PET membrane.
Fig. 2 shows results of evaluating Muse cell migration activity of the present extract.

### [Mode for Carrying Out the Invention]

The present invention is based on a study that researched the action of an extract of the present invention on migration of Muse cells isolated from mesenchymal stem cells derived from human adipocytes. Preferable embodiments of the present invention include, but not limited to, the embodiments presented below. In the present specification, an invention of "an agent for promoting migration of pluripotent stem cells containing an extract from inflamed tissues inoculated with vaccinia virus" subsumes both of the following inventions: an agent for promoting migration of pluripotent stem cells that is an extract from inflamed tissues inoculated with vaccinia virus; and an agent for promoting migration of pluripotent stem cells that is a preparation containing an extract from inflamed tissues inoculated with vaccinia virus.

(1) An agent for promoting migration of pluripotent stem cells containing an extract from inflamed tissues inoculated with vaccinia virus.
(2) The agent for promoting migration of pluripotent stem cells according to (1), wherein promotion of pluripotent stem cell migration is guidance of pluripotent stem cells to a damaged site of a living organism.
(3) The agent for promoting migration of pluripotent stem cells according to (1) or (2), wherein the pluripotent stem cells are SSEA-3-positive cells.
(4) The agent for promoting migration of pluripotent stem cells according to any one of (1) to (3), wherein the pluripotent stem cells are Muse cells.
(5) The agent for promoting migration of pluripotent stem cells according to any one of (1) to (4), wherein the inflamed tissues are skin tissues.
(6) The agent for promoting migration of pluripotent stem cells according to any one of (1) to (4), wherein the inflamed tissues are skin tissues of rabbits.
(7) The agent for promoting migration of pluripotent stem cells according to any one of (1) to (6), wherein the agent is an injectable preparation.
(8) The agent for promoting migration of pluripotent stem cells according to any one of (1) to (6), wherein the agent is an oral preparation.
(9) The agent for promoting migration of pluripotent stem cells according to (8), wherein the oral preparation is a solid preparation.
(10) The agent for promoting migration of pluripotent stem cells according to (9), wherein the solid preparation is a tablet.
(11) The agent for promoting migration of pluripotent stem cells according to (8), wherein the oral preparation is a liquid preparation.
(12) The agent for promoting migration of pluripotent stem cells according to any one of (1) to (6), wherein the agent is administered through a catheter.
(13) A use of an extract from inflamed tissues inoculated with vaccinia virus in the manufacture of an agent for promoting migration of pluripotent stem cells.
(14) The use according to (13), wherein promotion of pluripotent stem cell migration is guidance of pluripotent stem cells to a damaged site of a living organism.
(15) The use according to (13) or (14), wherein the pluripotent stem cells are SSEA-3-positive cells.
(16) The use according to any one of (13) to (15), wherein the pluripotent stem cells are Muse cells.
(17) The use according to any one of (13) to (16), wherein the inflamed tissues are skin tissues.
(18) The use according to any one of (13) to (16), wherein the inflamed tissues are skin tissues of rabbits.
(19) The use according to any one of (13) to (18), wherein the agent for promoting migration of pluripotent stem cells is an injectable preparation.
(20) The use according to any one of (13) to (18), wherein the agent for promoting migration of pluripotent stem cells is an oral preparation.
(21) The use according to (20), wherein the oral preparation is a solid preparation.
(22) The use according to (21), wherein the solid preparation is a tablet.
(23) The use according to (20), wherein the oral preparation is a liquid preparation.
(24) The use according to any one of (13) to (18), wherein the agent for promoting migration of pluripotent stem cells is administered through a catheter.
(25) A method for determining or evaluating an extract from inflamed tissues inoculated with vaccinia virus or a preparation containing the extract, wherein the action of promoting pluripotent stem cell migration is used as an index.
(26) The method according to (25), wherein the pluripotent stem cells are cultured cells.
(27) The method according to (25) or (26), wherein the pluripotent stem cells are SSEA-3-positive cells.
(28) The method according to any one of (25) to (27), wherein the pluripotent stem cells are Muse cells.
(29) The method according to any one of (25) to (28), wherein the inflamed tissues are skin tissues.
(30) The method according to any one of (25) to (28), wherein the inflamed tissues are skin tissues of rabbits.
(31) An extract from inflamed tissues inoculated with vaccinia virus or a preparation containing the extract, wherein the extract has been verified to satisfy the quality standard by the method as described in any one of (25) to (30).
(32) A method for verifying that the extract from inflamed tissues inoculated with vaccinia virus or the preparation containing the extract satisfies the quality standard by performing the determination or evaluation as described in any one of (25) to (30).
(33) An extract from inflamed tissues inoculated with vaccinia virus or a preparation containing the extract for use in the prevention, treatment, or progress inhibition of damage.
(34) The extract from inflamed tissues inoculated with vaccinia virus or the preparation containing the extract according to (33), wherein the prevention, treatment, or progress inhibition of damage is achieved by migration of pluripotent stem cells to a damaged site.
(35) The extract from inflamed tissues inoculated with vaccinia virus or the preparation containing the extract according to (34), wherein the pluripotent stem cells are SSEA-3-positive cells.
(36) The extract from inflamed tissues inoculated with vaccinia virus or the preparation containing the extract according to (34) or (35), wherein the pluripotent stem cells are Muse cells.
(37) The extract from inflamed tissues inoculated with vaccinia virus or the preparation containing the extract according to any one of (33) to (36), wherein the inflamed tissues are skin tissues.
(38) The extract from inflamed tissues inoculated with vaccinia virus or the preparation containing the extract according to any one of (33) to (36), wherein the inflamed tissues are skin tissues of rabbits.
(39) The extract from inflamed tissues inoculated with vaccinia virus or the preparation containing the extract according to any one of (33) to (38), wherein the extract or the preparation is administered by injection.
(40) The extract from inflamed tissues inoculated with vaccinia virus or the preparation containing the extract according to any one of (33) to (38), wherein the extract or the preparation is administered orally.
(41) The extract from inflamed tissues inoculated with vaccinia virus or the preparation containing the extract according to any one of (33) to (38), wherein the extract or the preparation is administered through a catheter.
(42) A method for prevention, treatment, or progress inhibition of damage by administering an effective amount of an extract from inflamed tissues inoculated with vaccinia virus or a preparation containing the extract to a patient in need of treatment.
(43) The method according to (42), wherein the prevention, treatment, or progress inhibition of damage is achieved by migration of pluripotent stem cells to a damaged site in a living organism.
(44) The method according to (43), wherein the pluripotent stem cells are SSEA-3-positive cells.
(45) The method according to (43) or (44), wherein the pluripotent stem cells are Muse cells.
(46) The method according to any one of (42) to (45), wherein the inflamed tissues are skin tissues.
(47) The method according to any one of (42) to (45), wherein the inflamed tissues are skin tissues of rabbits.
(48) The method according to any one of (42) to (47), wherein the administration is performed by injection.
(49) The method according to any one of (42) to (47), wherein the administration is performed orally.
(50) The method according to any one of (42) to (47), wherein the administration is performed through a catheter.

The present extract is an extract containing a non-proteinous active substance extracted and separated from the inflamed skin tissues of rabbits by the inoculation of vaccinia virus. Although the present extract is liquid in an extracted state, it is also possible to make into a solid by means of drying. a preparation containing the present extract (hereinafter, it will be sometimes called "the present preparation") is very useful as a drug. In a specific product as the present preparation which is manufactured and distributed by the applicant, there is "a preparation containing an extract from inflamed skins of rabbits inoculated with vaccinia virus" (trade name: NEUROTROPIN [registered trademark]; hereinafter, it will be referred to as "NEUROTROPIN"). In NEUROTROPIN, there are injection and tablet and both belong to an ethical drug.

Indications of NEUROTROPIN injection are "low back pain, cervicobrachial syndrome, symptomatic neuralgia, itchiness accompanied by skin diseases (eczema, dermatitis, urticaria), allergic rhinitis and sequelae of subacute myelo-optico-neuropathy (SMON) such as coldness, paresthesia and pain". Indications of NEUROTROPIN tablet are "postherpetic neuralgia, low back pain, cervicobrachial syndrome, periarthritis scapulohumeralis and osteoarthritis". NEUROTROPIN preparation has been created and developed as a drug by the applicant. NEUROTROPIN preparation has been appreciated for its excellent advantage for efficacy and safety, sold for many years and established a firm position in the Japanese pharmaceutical market.

The extract from inflamed tissues inoculated with vaccinia virus used in the present invention can be obtained by the following manner: inflamed tissues inflamed by the inoculation with vaccinia virus is crushed; an extraction solvent is added to remove the tissue fragments; then deproteinization is carried out; the deproteinized solution is adsorbed onto an adsorbent; and then the active ingredient is eluted. for example, according to the following process.
(A) Inflamed skin tissues of rabbits, mice or the like by the inoculation with vaccinia virus are collected, and the inflamed tissues are crushed. To the crushed tissue an extraction solvent such as water, phenolated water, physiological saline or phenol-added glycerin water is added. Then, the mixture is filtered or centrifuged to obtain an extraction liquid (filtrate or supernatant).
(B) The pH of the extraction liquid is adjusted to be acidic and the liquid is heated for deproteinization. Then, the deproteinized solution is adjusted to be alkaline, heated, and then filtered or centrifuged.
(C) The obtained filtrate or supernatant is made acidic and adsorbed onto an adsorbent such as activated carbon or kaolin.
(D) To the adsorbent, an extraction solvent such as water is added, the pH is adjusted to alkaline, and the adsorbed component is eluted to obtain the extract from inflamed tissues inoculated with vaccinia virus. Subsequently, as desired, the eluate may be evaporated to dryness under reduced pressure or freeze-dried to give a dried material.

As for animals in order to obtain the inflamed tissues by the inoculation of vaccinia virus, various animals that is infected with vaccinia virus such as rabbits, cows, horses, sheep, goats, monkeys, rats or mice can be used, and preferred inflamed tissues are inflamed skin tissues of rabbits. With regard to a rabbit, any rabbit may be used so far as it belongs to Lagomorpha. Examples thereof include Oryctolagus cuniculus, domestic rabbit (domesticated Oryctolagus cuniculus), hare (Japanese hare), mouse hare and snowshoe hare. Among them, it is appropriate to use domestic rabbit. In Japan, there is family rabbit called "Kato" which has been bred since old time and frequently used as livestock or experimental animal and it is another name of domestic rabbit. There are many breeds in domestic rabbit and the breeds being called Japanese white and New Zealand white are advantageously used.

Vaccinia virus used herein may be in any strain. Examples thereof include Lister strain, Dairen strain, Ikeda strain, EM-63 strain and New York City Board of Health strain.

As to basic extracting steps (A) to (D) of the above-described for the present extract can be carried out in more detail, the following steps are used for example.

### About step (A):

The inflamed skin tissues of rabbits by the intradermal inoculation of vaccinia virus are collected. The collected skin tissues are washed and disinfected using a phenol solution, etc. This inflamed skin tissues are crushed and an extraction solvent in 1- to 5-fold thereof by volume is added thereto. Here, the term "crush" means to finely break down into minces using a mincing machine or the like. As to the extraction solvent, there may be used distilled water, physiological saline, weakly acidic to weakly basic buffer, etc. and bactericidal/antiseptic agent such as phenol, stabilizer such as glycerin, salts such as sodium chloride, potassium chloride or magnesium chloride, etc. may be appropriately added thereto. At that time, it is also possible that the cell tissue is destroyed by a treatment such as freezing/melting, ultrasonic wave, cell membrane dissolving enzyme or surfactant so as to make the extraction easier. The resulting suspension is allowed to stand for 5 to 12 days. During that period, the suspension may be heated at 30 to 45°C with or without appropriate stirring. The resulting liquid is subjected to a treatment for separating into solid and liquid (filtered or centrifuged, etc.) to remove the tissue fragments whereupon a crude extract (filtrate or supernatant) is obtained.

### About step (B)

The crude extract obtained in step (A) is subjected to a deproteinizing treatment. The deproteinization may be carried out by a known method which has been usually conducted and a method such as heating treatment, treatment with a protein denaturant (such as acid, base, urea, guanidine or an organic solvent including acetone), isoelectric precipitation or salting-out may be applied. After that, a common method for the removal of insoluble matters such as filtration using filter paper (such as cellulose or nitrocellulose), glass filter, Celite or Seitz filter, ultrafiltration or centrifugation is conducted to give a filtrate or a supernatant wherefrom the separated insoluble protein is removed.

### About step (C)

The filtrate or supernatant obtained in step (B) is adjusted to acidic or, preferably, to pH 3.5 to 5.5 to conduct an operation of adsorbing with an adsorbent. Examples of the usable adsorbent include activated carbon and kaolin. An adsorbent is added to the extract followed by stirring or the extract is passed through a column filled with an adsorbent so that the active ingredient can be adsorbed with the adsorbent. When an adsorbent is added to the extract, the adsorbent with which the active ingredient is adsorbed can be obtained by means of filtration, centrifugation, etc. to remove the solution.

### About step (D)

For elution (desorption) of the active ingredient from the adsorbent obtained in step (C), an elution solvent is added to said adsorbent and adjusted to basic or, preferably, to pH 9 to 12, elution is conducted at room temperature or with suitable heating, or with stirring, and then the adsorbent is removed by a common method such as filtration or centrifugation. As to the extraction solvent used therefore, there may be used a basic solvent such as water, methanol, ethanol, isopropanol or the like adjusted to basic pH or an appropriate mixed solvent thereof and preferably, water adjusted to pH 9 to 12 may be used. Amount of the extracting solvent may be appropriately set. In order to use the eluate obtained as such as a drug substance, the pH is appropriately adjusted to nearly neutral or the like whereby an extract from inflamed skins of rabbits inoculated with vaccinia virus (the present extract) can be finally obtained.

Since the present extract is liquid at the stage of being prepared, it is also possible that said extract is appropriately concentrated or diluted to make into a desired concentration. When a preparation is manufactured from the present extract, it is preferred to apply a sterilizing treatment with heating. For making into an injectable preparation, it is possible to add sodium chloride or the like so as to prepare a solution being isotonic to physiological saline. It is also possible that the present extract is administered in a liquid or gel state. Furthermore, the present extract may be subjected to an appropriate operation such as concentration to dryness to prepare a solid preparation for oral administration such as a tablet. Specific methods for the manufacture of solid preparation for oral administration from the present extract are disclosed in the specifications of Japanese Patent Nos. 3,818,657 and 4,883,798. The present preparation includes an injectable preparation, a solid preparation for oral administration, etc. prepared as such. In addition, the present preparation may be topically applied where to make Muse cells migrate by use of a catheter.

The method for administering a pharmaceutically effective amount to a patient in need of treatment is not particularly limited and may be suitably selected depending on the purpose of treatment. Examples of the method include oral administration, subcutaneous administration, intramuscular administration, intravenous administration, and transdermal administration. In order to make the pluripotent stem cell accumulate at a damaged site, direct administration to the damaged site through a catheter or the like is desirable. The dose may be suitably determined depending on the type of the extract from inflamed tissues inoculated with vaccinia virus. The dose that is approved in the commercially available preparation is principally 16 NU per day by oral administration and 3.6 to 7.2 NU per day by injection. However, the dose may be appropriately increased or decreased depending on the type of disease, degree of seriousness, individual difference in the patients, method of administration, period of administration and the like (NU: Neurotropin unit. Neurotropin unit is defined by ED50 value of analgesic effect measured by a modified Randall-Selitto method using SART-stressed mice that are chronic stressed animals showing a lowered pain threshold than normal animals. One NU indicates the activity of 1 mg of analgesic ingredients in Neurotropin preparations when the ED50 value is 100 mg/kg of the preparation).

Hereinafter, examples of methods for producing the present extract as well as clinical evaluation concerning novel pharmacological activity of the extract, the promoting migration of pluripotent stem cells, are described. The present invention is not intended to be limited to the descriptions in Examples.

### [Examples]

### Example 1 (Manufacture of the present extract)

Skins of healthy adult rabbits were inoculated with vaccinia virus intradermally and the inflamed skins were cut and collected. The collected skins were washed and disinfected by a phenol solution, an excessive phenol solution was removed and the residue was crushed. A phenol solution was added thereto and mixed therewith and the mixture was allowed to stand for 3 to 7 days, and further heated at 35 to 40°C together with stirring for 3 to 4 days. After that, an extracted solution obtained by a solid-liquid separation was adjusted to pH 4.5 to 5.2 with hydrochloric acid, heated at 90 to 100°C for 30 minutes and filtered to remove protein. The filtrate was adjusted to pH 9.0 to 9.5 with sodium hydroxide, heated at 90 to 100°C for 15 minutes and subjected to a solid-liquid separation.

The resulting deproteinized solution was adjusted to pH 4.0 to 4.3 with hydrochloric acid, activated carbon in an amount of 2% to the mass of the deproteinized solution was added thereto and the mixture was stirred for 2 hours and subjected to the solid-liquid separation. Water was added to the collected activated carbon followed by adjusting to pH 9.5 to 10 with sodium hydroxide and the mixture was stirred at 60°C for 90 to 100 minutes and centrifuged to give a supernatant. Water was added again to the activated carbon precipitated upon the centrifugation followed by adjusting to pH 10.5 to 11 with sodium hydroxide and the mixture was stirred at 60°C for 90 to 100 minutes and centrifuged to give a supernatant. Both supernatants were combined and neutralized with hydrochloric acid to give the present extract.

### Example 2 (Test method and test results)

Below is the method of a pharmacological test conducted for evaluating the action of the present extract obtained in Example 1 to activate migration of Muse cells, as well as the test results.

### Test Example 1 Evaluation by Boyden chamber method

### Cells and reagents

Mesenchymal stem cells derived from human adipose tissue (Lonza) were cultured in a Dulbecco modified Eagle medium (DMEM, Gibco) containing 15% fetal bovine serum (FBS) and 0.1 mg/mL kanamycin sulfate (Gibco) in a CO₂ incubator at 37°C.

### Measurement of chemotactic ability of Muse cells

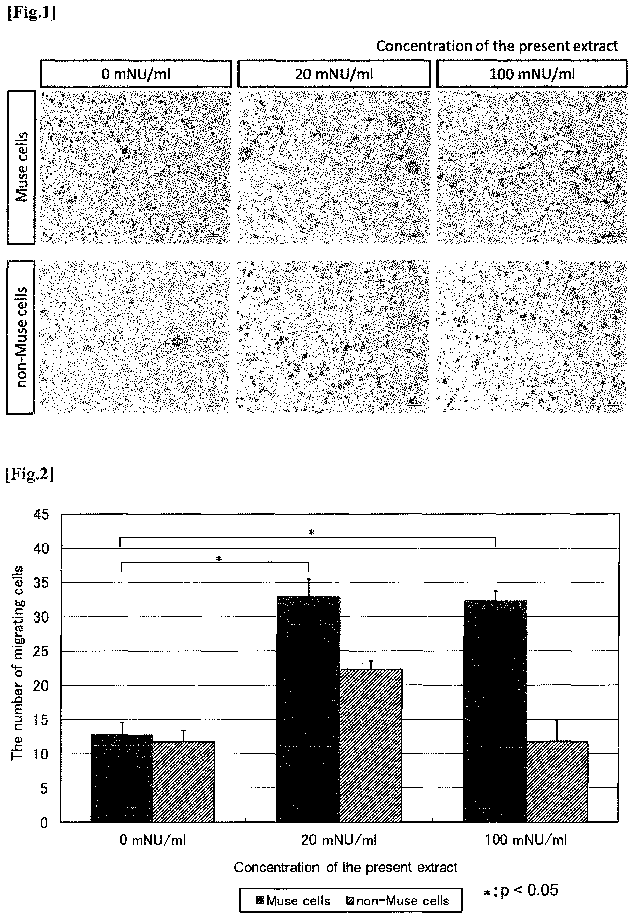

Muse cells and non-Muse cells were separated from each other by FACS (fluorescence activated cell sorting) the day before measurement, followed by overnight culturing under conditions of 37°C and 5% CO₂. On the day of measurement, a culture insert (upper chamber) of a Corning BioCoat Matrigel (registered trademark; the same applies hereinafter) Invasion Chamber (24 wells, 8.0 µm, BD Bioscience) was immersed in an assay medium, followed by incubation in a 5% CO₂ incubator at 37°C for 2 hours. After the 2 hours, a medium containing the present extract at a concentration of 0, 20, or 100 mNU/mL was added to a companion plate (lower chamber) in an amount of 750 µL per well. The Muse cells and the non-Muse cells separated the day before were counted by a trypan blue exclusion test, followed by suspending the Muse cells and the non-Muse cells each in a separate medium containing 10% FBS in an amount of 5 × 10⁴ cells/500 µL. The resulting cell suspension was added to the culture insert in an amount of 500 µL per well, and then the Corning BioCoat Matrigel Invasion Chamber was left still standing under conditions of 37°C and 5% CO₂. After being left for 24 hours, cells that had not passed through a PET membrane and remained on an upper surface of the culture insert were scraped off the culture insert with a cotton swab. Cells that had passed through the PET membrane and migrated to a lower surface of the culture insert were stained with a Diff-Quik kit (Sysmex Corporation). The PET membrane was air dried. Then, the cells below the PET membrane were observed and counted under 200-time magnification, four independent fields of view per membrane. The average of the cell counts observed in the four fields of view was defined as the number of migrated cells for the membrane. Fig. 1 shows stained images of migrated cells that passed through the PET membrane. Cells, which are naturally purple, are stained with a light color in the stained images (the black points are not cells).

### Statistical analysis

The two groups were compared to each other by Student's t-test. When the relationship p < 0.05 was satisfied, the result was considered to be "significant". The test was repeated three times, and the average cell count and the standard error were determined. All of these three tests gave the same results, and typical data among these is shown.

### Test results

The group of Muse cells showed a significant increase in the number of migrated cells at a concentration of the present extract of either 20 mNU/mL or 100 mNU/mL, proving the action of the present extract to promote migration of Muse cells. The group of non-Muse cells showed no significant increase in the number of migrated cells. These results indicate that the present extract specifically promotes migration of Muse cells. Fig. 2 shows an example of the results. In the drawing, the abscissa shows different concentrations of the present extract and the ordinate shows the number of Muse cells that were made migrate by the action of the present extract.

### Test Example 2 Evaluation by cell mobility analysis technology (TAXIScan Technology)

### Cells and reagents

Mesenchymal stem cells derived from human bone marrow in a concentration of 1 × 10⁷ cells/mL were prepared using an FACS buffer containing Dulbecco PBS, 0.5% bovine serum albumin, and 2 mM EDTA (ethylenediaminetetraacetic acid). Thereto, an anti-SSEA-3 antibody (diluted 400 times) was added as a primary antibody. Reaction was allowed to proceed on ice for 1 hour while being suspended at appropriate times, followed by centrifugation for supernatant removal. Then, the addition and removal of an FACS buffer were repeated three times for rinsing. Thereto, an FITC-labeled anti-rat-IgM antibody (diluted 100 times) was added as a secondary antibody. Subsequently, in the same manner as above, reaction was allowed to proceed on ice for 1 hour and then addition and removal of an FACS buffer were repeated three times for rinsing.

### Measurement of chemotactic ability of Muse cells

SSEA-3-positive cells were separated by FACS the day before measurement, followed by overnight culturing under conditions of 37°C and 5% CO₂. On the day of measurement, the cultured cells were collected and then suspended in a chemotactic-ability-measurement medium containing 25 mM HEPES (2-[4-(2-hydroxyethyl)piperazin-1-yl]ethanesulfonic acid). The resulting cell suspension was injected into each well of an EZ-TAXIScan (registered trademark; the same applies hereinafter) holder chamber (GE Healthcare), and the cells were aligned properly in front of a terrace by a water-level difference method. The terrace had a depth of 6 µm. The EZ-TAXIScan holder chamber was placed in an incubator under conditions of 37°C and 5% CO₂. Then, the EZ-TAXIScan holder chamber was taken out of the incubator and attached to a main body plate of the EZ-TAXIScan (GE Healthcare). To each well on a sample side, 50 mNU/mL of the present extract or 2 µM of sphingosine-1-phosphate (S1P) as a positive control was added. Each well was photographed every 10 minutes for measuring chemotactic ability with time. S1P is a lipid mediator derived from sphingolipid and regulates survival, growth, differentiation, movement, and the like of cells via an S1P receptor. It was found that S1P has action of promoting migration of Muse cells (WO 2014/133170).

### Test results

The group that received the present extract showed movement of Muse cells toward where the present extract was added, with multiple cells observed to have even moved to the outside of the chamber. The same movement of Muse cells was observed in the positive control group, which received S1P.

### [Industrial Applicability]

These results have proven the action of the present extract to promote migration of Muse cells. Thus, it has been suggested that administration of the present extract or a preparation containing the extract is a potentially effective method for facilitating damage treatment.

## Claims

1. An agent for promoting migration of pluripotent stem cells containing an extract from inflamed tissues inoculated with vaccinia virus.

2. The agent for promoting migration of pluripotent stem cells according to claim 1, wherein promotion of pluripotent stem cell migration is guidance of pluripotent stem cells to a damaged site of a living organism.

3. The agent for promoting migration of pluripotent stem cells according to claim 1 or 2, wherein the pluripotent stem cells are SSEA-3-positive cells.

4. The agent for promoting migration of pluripotent stem cells according to any of claims 1 to 3, wherein the pluripotent stem cells are Muse cells.

5. The agent for promoting migration of pluripotent stem cells according to any of claims 1 to 4, wherein the inflamed tissues are skin tissues.

6. The agent for promoting migration of pluripotent stem cells according to any of claims 1 to 4, wherein the inflamed tissues are skin tissues of rabbits.

7. The agent for promoting migration of pluripotent stem cells according to any of claims 1 to 6, wherein the agent is an injectable preparation.

8. The agent for promoting migration of pluripotent stem cells according to any of claims 1 to 6, wherein the agent is an oral preparation.

9. The agent for promoting migration of pluripotent stem cells according to any of claims 1 to 6, wherein the agent is administered through a catheter.

10. A use of an extract from inflamed tissues inoculated with vaccinia virus in the manufacture of an agent for promoting migration of pluripotent stem cells.

11. The use according to claim 10, wherein promotion of pluripotent stem cell migration is guidance of pluripotent stem cells to a damaged site of a living organism.

12. The use according to claim 10 or 11, wherein the pluripotent stem cells are SSEA-3-positive cells.

13. The use according to any of claims 10 to 12, wherein the pluripotent stem cells are Muse cells.

14. The use according to any of claims 10 to 13, wherein the inflamed tissues are skin tissues.

15. The use according to any of claims 10 to 13, wherein the inflamed tissues are skin tissues of rabbits.

16. The use according to any of claims 10 to 15, wherein the agent for promoting migration of pluripotent stem cells is an injectable preparation.

17. The use according to any of claims 10 to 15, wherein the agent for promoting migration of pluripotent stem cells is an oral preparation.

18. The use according to any of claims 10 to 15, wherein the agent for promoting migration of pluripotent stem cells is administered through a catheter.

19. A method for determining or evaluating an extract from inflamed tissues inoculated with vaccinia virus or a preparation containing the extract, wherein the action of promoting pluripotent stem cell migration is used as an index.

20. The method according to claim 19, wherein the pluripotent stem cells are cultured cells.

21. The method according to claim 19 or 20, wherein the pluripotent stem cells are SSEA-3-positive cells.

22. The method according to any of claims 19 to 21, wherein the pluripotent stem cells are Muse cells.

23. The method according to any of claims 19 to 22, wherein the inflamed tissues are skin tissues.

24. The method according to any of claims 19 to 22, wherein the inflamed tissues are skin tissues of rabbits.

25. An extract from inflamed tissues inoculated with vaccinia virus, wherein the extract has been verified to satisfy the quality standard by the method as described in any of claims 19 to 24.

26. A method for verifying that the extract from inflamed tissues inoculated with vaccinia virus satisfies the quality standard by performing the determination or evaluation as described in any of claims 19 to 24.
